(19) Europäisches Patentamt
European Patent Office
Office européen des brevets

(11) Veröffentlichungsnummer: **0 137 993**
**B1**

(12) # EUROPÄISCHE PATENTSCHRIFT

(45) Veröffentlichungstag der Patentschrifft: **17.01.90**

(51) Int. Cl.⁴: **C 07 D 487/04**, A 61 K 31/55, C 07 D 233/90 // (C07D487/04, 243:00, 235:00)

(21) Anmeldenummer: **84110215.5**

(22) Anmeldetag: **28.08.84**

(54) **Neue 11-Piperazinyl-5H-imidazo 2,1-c 1,4 benzodiazepine, Verfahren zu ihrer Herstellung und Zwischenprodukte und erstere enthaltende Arzneimittel.**

(30) Priorität: **03.09.83 DE 3331858**

(43) Veröffentlichungstag der Anmeldung: **24.04.85 Patentblatt 85/17**

(45) Bekanntmachung des Hinweises auf die Patenterteilung: **17.01.90 Patentblatt 90/03**

(84) Bennante Vertragsstaaten: **AT BE CH DE FR GB IT LI LU NL SE**

(56) Entgegenhaltungen:

**SYNTHESIS, Nr. 4, April 1981, Seiten 321-324, Georg Thieme Verlag, Stuttgart, New York, US; G. STEFANCICH et al.: "Research on nitrogen heterocyclic compounds; XIII. Synthesis of 5H-imidazo 2,1-c 1,4 benzodiazepine, a novel tricyclic ring system"**

**Die Akte enthält technische Angaben, die nach dem Eingang der Anmeldung eingereicht wurden und die nicht in dieser Patentschrift enthalten sind.**

(73) Patentinhaber: **BOEHRINGER INGELHEIM KG**
**Postfach 200**
**D-6507 Ingelheim am Rhein (DE)**
(84) Bennante Vertragsstaaten: **BE CH DE FR IT LI LU NL SE AT**

(73) Patentinhaber: **BOEHRINGER INGELHEIM INTERNATIONAL GmbH**
**Postfach 20**
**D-6507 Ingelheim am Rhein (DE)**
(84) Bennante Vertragsstaaten: **GB**

(72) Erfinder: **Walther, Gerhard, Dr.**
**Pfarrer-Heberer-Strasse 37**
**D-6530 Bingen/Rhein (DE)**
Erfinder: **Schneider, Claus, Dr.**
**Albrecht-Dürer-Strasse 19**
**D-6507 Ingelheim/Rhein (DE)**
Erfinder: **Weber, Karl-Heinz, Dr.**
**Kaiser-Karl-Strasse 11**
**D-6535 Gau-Algesheim (DE)**
Erfinder: **Fügner, Armin, Dr.**
**Im Hippel 31**
**D-6535 Gau-Algesheim (DE)**

EP 0 137 993 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die Erfindung betrifft neue 11-Piperazinyl-5H-imidazo[2,1-c][1,4]benzodiazepine der allgemeinen Formel

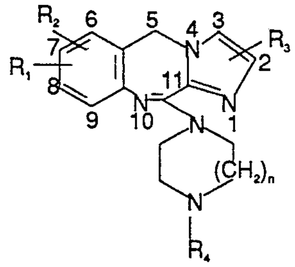

(I)

und deren Säureadditionssalze.

In dieser Formel bedeuten:

$R_1$ und $R_2$, die gleich oder verschieden sein können, Wasserstoff, Fluor, Chlor, Brom, Trifluormethyl, Hydroxy, Nitro, Amino, Cyano, Methylsulfonamido, $C_{1-4}$ Alkyl, $C_{1-3}$ Alkoxy, $C_{1-3}$ Alkylthio oder $C_{2-4}$ Acylamino,

$R_3$ Wasserstoff, Fluor, Chlor, Brom oder $C_{1-3}$ Alkyl,

$R_4$ Wasserstoff, $C_{1-4}$ Alkyl, $C_{2-4}$ Hydroxyalkyl, $C_{1-2}$ Alkoxy-$C_{2-4}$ alkyl, $C_{3-6}$ Alkenyl, $C_{3-6}$ Alkinyl, $C_{3-7}$ Cycloalkyl, $C_{3-7}$ Cycloalkyl-$C_{1-3}$ alkyl oder Phenyl-$C_{1-3}$ Alkyl, wobei der Phenylring gegebenenfalls durch Fluor, Chlor, Brom, Methyl, Methoxy, Hydroxy oder Trifluormethyl substituiert sein kann, und

n eine der Zahlen 1 oder 2.

Die Erfindung betrifft ferner die Zwischenprodukte der allgemeinen Formeln II und IV, Verfahren zur Herstellung der Endprodukte und diese enthaltende Arzneimittel.

Die Verbindungen der allgemeinen Formel I zeigen wertvolle pharmakologische Wirkungen. Im Vordergrund steht insbesondere eine starke antiallergische Komponente. Einige der neuen Verbindungen zeigen ferner eine analgetische, Antihistamin- oder neuroleptische Wirkung.

Bevorzugt sind solche Verbindungen der Formel I, in denen $R_1$ und $R_2$ in 7- bzw. 8-Stellung des Moleküls Wasserstoff oder ein Halogenatom und $R_3$ Wasserstoff bedeutet und $R_4$ für einen der oben angegebenen Substituenten steht, vorzugsweise für Wasserstoff oder einen Methylrest. Die Herstellung der neuen Verbindungen kann in an sich bekannter. Weise dadurch erfolgen, daß man

a) eine Verbindung der allgemeinen Formel

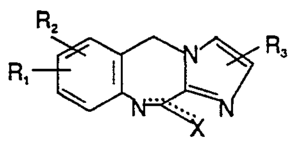

(II)

worin $R_1$, $R_2$ und $R_3$ die oben angegebene Bedeutung besitzen und X eine abspaltbare Gruppe bedeutet, mit einem Piperazin der allgemeinen Formel

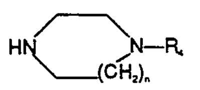

(III)

worin $R_4$ und n die oben angegebene Bedeutung besitzen, umsetzt.

Als abspaltbarer Rest kommt in Frage eine Alkoxy- oder Alkylmercaptogruppe mit 1-4 Kohlenstoffatomen, ein Chlor- oder Bromatom sowie = 0 oder = S.

b) eine Verbindung der allgemeinen Formel

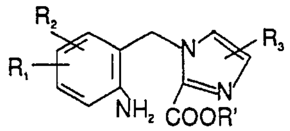

(IV)

worin $R_1$, $R_2$ und $R_3$ die oben angeführte Bedeutung besitzen und R' einen niederen Alkylrest, bevorzugt einen Methyl- oder Äthylrest bedeutet mit einem Piperazin der allgemeinen Formel III umsetzt, oder

c) eine Verbindung der allgemeinen Formel

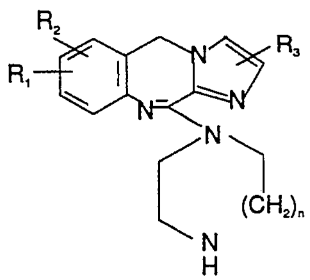

(V)

worin $R_1$, $R_2$, $R_3$ und n die oben angeführte Bedeutung besitzen mit einer Verbindung der allgemeinen Formel

$R_4$-Z (VI)

worin $R_4$ die oben angeführten Bedeutungen mit Ausnahme von Wasserstoff besitzt und Z eine leicht abspaltbare Gruppe wie z. B. ein Halogenatom, einen Tosyloxyrest oder den Mesyloxyrest bedeutet, umsetzt.

Die Reaktion zwischen den Verbindungen der allgemeinen Formeln II und III wird im Falle X = Chlor, Brom oder Jod bevorzugt in Gegenwart eines tertiären Amins, z. B. Triäthylamin, als säurebindendem Agens durchgeführt. Es ist aber auch möglich, einen Überschuß des Amins der Formel III ohne weiteres Lösungsmittel zu verwenden. Falls X eine Alkoxy- oder Alkylmercaptogruppe bedeutet, ist die Hilfsbase entbehrlich. Geeignete Lösungsmittel sind Toluol oder Chlorbenzol. Die Umsetzungen werden bevorzugt bei Temperaturen von 100-150°C durchgeführt, gelingen jedoch auch bei niederen Temperaturen bei entsprechend längerer Reaktionszeit.

Die Umsetzung solcher Verbindungen der Formel II, in denen X ein Sauerstoffatom bedeutet, mit einem Amin der Formel III wird unter den vorstehend angegebenen Bedingungen in Gegenwart von Titantetrachlorid durchgeführt. Hierbei hat sich als besonders vorteilhaft erwiesen, wenn man als Lösungsmittel oder Cosolvens Anisol verwendet, das mit dem Titantetrachlorid einen löslichen Komplex bildet.

Die Umsetzung von Verbindungen der Formel IV mit dem Piperazin der allgemeinen Formel III gelingt ebenfalls in Gegenwart von Titantetrachlorid unter den oben beschriebenen Reaktionsbedingungen.

Die Alkylierung von Verbindungen der allgemeinen Formel V mit Verbindungen der allgemeinen Formel VI erfolgt in einem geeigneten inerten Lösungsmittel, beispielsweise Acetonitril, einem Alkohol oder einem Keton unter Rückflußtemperatur des Reaktionsgemisches und gegebenenfalls in Gegenwart einer organischen oder anorganischen Base.

Je nach der eingesetzten Ausgangsverbindung kann sich eine der vorstehend beschriebenen Verfahrensvarianten als besonders günstig zur Herstellung eines bestimmten Endprodukts erweisen.

Die Herstellung der als Ausgangsstoffe verwendeten Aminoester der allgemeinen Formel IV kann entsprechend dem nachstehenden Reaktionsschema erfolgen:

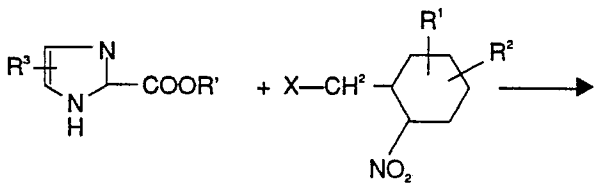

(VII) (VIII)

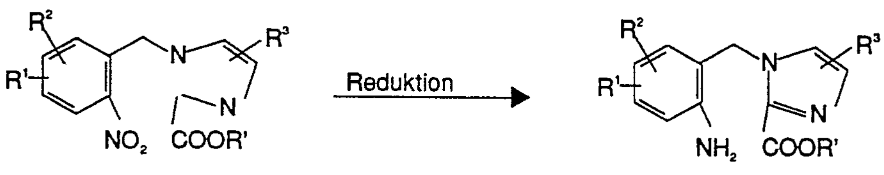


(IX) (IV)

Die Verbindungen der Formel VII sind bekannt oder können analog in der Literatur beschriebener Verfahren [z. B. K. L. Kirk, J. Org. Chem. *43*/22, 4 381-4 383 (1978)] dargestellt werden.

Die Alkylierung von Verbindungen der Formel VII erfolgt in an sich bekannter Weise durch Umsetzung mit Verbindungen der Formel VIII (X = z. B. ein Halogenatom, bevorzugt ein Chlor- oder Bromatom, die Tosyloxy- oder Mesyloxygruppe). Bevorzugt wird die Umsetzung in Dimethylformamid in Gegenwart von wasserfreiem Kaliumcarbonat bei einer Reaktionstemperatur von 80 - 120°C durchgeführt.

Die Nitroverbindungen der Formel IX können anschließend durch Reduktion, z. B. durch katalytische Hydrierung in Gegenwart eines geeigneten Katalysators, wie Raney-Nickel, mit Zinn-(II)-chlorid und Salzsäure in wäßrigem Alkohol oder Ammoniumpolysulfid, in die entsprechenden Aminoverbindungen der Formel IV umgewandelt werden.

Verbindungen der Formel II (X = O) können in an sich bekannter Weise durch Ringschluß von Aminoestern der Formel IV (R' = $C_{1-4}$ Alkyl) z. B. in Gegenwart von Dimethylsulfinyl-Natrium in einem geeigneten Lösungsmittel, wie Dimethylsulfoxyd, oder in Gegenwart eines Alkalialkoholats, z. B. Kalium-tert.-butylat, in einem inerten Lösungsmittel, wie Xylol, hergestellt werden. Die Darstellung von Verbindungen der Formel II (X = O) gelingt aber auch in saurer Reaktionslösung, z. B. durch Erhitzen einer Verbindung der Formel IV in Eisessig, gegebenenfalls unter Zugabe von katalytischen Mengen p-Toluolsulfonsäure. Eine weitere Alternative zur Herstellung von Verbindungen der Formel II (X = O) besteht im Ringschluß von Aminosäuren der Formel IV mit freier Carboxylgruppe, z. B. mit Dicyclohexylcarbodiimid in einem geeigneten Lösungsmittel wie Tetrahydrofuran.

Die Thioamide der Formel II (Y = S) werden durch Umsetzung der entsprechenden Amide II (Y = O), z. B. mit Phosphorpentasulfid, in einem wasserfreien basischen Lösungsmittel, wie Pyridin, dargestellt.

Die Verbindungen der Formel II (X = -SR, -OR oder Halogen) können durch Umsetzung von Verbindungen der Formel II (X = O bzw. = S) mit herkömmlichen Reagenzien erhalten werden.

Die Verbindungen der Formel IV mit freier Carboxylgruppe können durch Hydrolyse von Carbonsäureestern der Formel IV (R' = Alkyl), z. B. mit Natronlauge in Äthanol, erhalten werden.

Die nach den verschiedenen Verfahren erhaltenen Reaktionsprodukte werden mit Hilfe bekannter Labormethoden isoliert. Gegebenenfalls können die so erhaltenen Rohprodukte noch unter Verwendung besonderer Methoden, z. B. durch Säulenchromatographie, gereinigt werden, ehe man sie in der Basen oder geeigneter Salze kristallisiert. Zur Herstellung von Säureadditionssalzen werden insbesondere solche Säuren verwendet, die zur Bildung von therapeutisch verwertbaren Salzen geeignet sind, wie Halogenwasserstoffsäuren, Schwefelsäure, Phosphorsäure, Salpetersäure, Cyclohexylsulfaminsäure, Zitronensäure, Weinsäure, Ascorbinsäure, Maleinsäure, Ameisensäure, Salicylsäure oder Methan- oder Toluolsulfonsäure und dergleichen.

Die Verbindungen nach der Erfindung können in verschiedenen pharmazeutischen Zusammensetzungen angewendet werden.

Die neuen Verbindungen sind starke Antiallergica und übertreffen die Wirkung der bekannten Handelsprodukte

Ketotifen und Promethazin im PCA-Test (GOOSE et al, Immunology *16*, 749 (1969) um das Zehn- bis über Dreihundertfache. Sie sind im Gegensatz zu dem bekannten Handelsprodukt Intal® auch oral wirksam.

Geeignete Anwendungsformen sind beispielsweise Tabletten, Kapseln, Zäpfchen, Lösungen, Säfte, Emulsionen, Aerosole oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker sowie ein geschmacksverbesserndes Mittel, z. B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Äthylenoxid, oder Schutzstoffen wie p-Hydroxybenzoate, enthalten.

Injektionslösungen werden in üblicher Weise, z. B. unter Zusatz von Konservierungsmitteln wie p-Hydroxybenzoaten, oder Stabilisatoren, wie Alkalisalzen der Äthylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Für das Verfahren benötigte Ausgangsstoffe können beispielsweise auf folgendem Weg erhalten werden:

**11-Methylthio-5H-imidazo[2,1-c][1,4]benzodiazepin**

a) *1-(2-Nitrobenzyl)-imidazol-2-carbonsäureäthylester*

Eine Mischung von 14 g (0,1 Mol) Imidazol-2-carbonsäureäthylester, 18,9 g (0,11 Mol) o-Nitrobenzylchlorid, 13,8 g (0,1 Mol) wasserfreiem Kaliumcarbonat und 100 ml Dimethylformamid wird 2 Stunden unter Rühren auf 100°C erhitzt. Anschließend wird das Lösungsmittel im Vakuum weitgehend abdestilliert, der Rückstand mit 100 ml Wasser versetzt und mit Essigester ausgeschüttelt. Die organische Phase wird noch zweimal mit Wasser gewaschen, über wasserfreiem Natriumsulfat getrocknet und eingedampft. Der kristalline Rückstand wird in Äther suspendiert und abgesaugt. Man erhält 23,5 g (85,4 % d. Th.) 1-(2-Nitrobenzyl)-imidazol-2-carbonsäureäthylester vom Fp. 107 - 111°C.

In analoger Weise erhält man aus den entsprechenden o-Nitrobenzylhalogeniden und Imidazol-2-carbonsäureäthylestern die folgenden Verbindungen:

1-(2-Nitro-4-chlor-benzyl)-imidazol-2-carbonsäureäthylester;
1-(2-Nitro-5-methyl-benzyl)-imidazol-2-carbonsäureäthylester; Fp. 105 - 107°C (Essigester/Benzin)
1-(2-Nitro-5-chlor-benzyl)-imidazol-2-carbonsäureäthylester;
1-(2-Nitro-5-methoxy-benzyl)-imidazol-2-carbonsäureäthylester;
1-(2-Nitro-4-fluor-benzyl)-imidazol-2-carbonsäureäthylester.

b) *1-(2-Aminobenzyl)-imidazol-2-carbonsäureäthylester*

49,6 g (0,18 Mol) 1-(2-Nitrobenzyl)-imidazol-2-carbonsäureäthylester werden in 500 ml Tetrahydrofuran bei Rumtemperatur und 5 bar über Raney-Nickel hydriert. Nach Aufnahme der theoretischen Wasserstoffmenge wird der Katalysator abgesaugt und die Lösung eingedampft. Man erhält 37,7 g (85,4 % d. Th.) 1-(2-Aminobenzyl)-imidazol-2-carbonsäureäthylester vom Fp. 104 - 106°C (Fp. aus Toluol 105 - 107°C).

In analoger Weise können aus den entsprechenden Nitroverbindungen die nachstehenden Aminoverbindungen dargestellt werden.

1-(2-Amino-4-chlor-benzyl)-imidazol-2-carbonsäureäthylester;
1-(2-Amino-5-methyl-benzyl)-imidazol-2-carbonsäureäthylester; Fp. 94 - 96°C (Toluol/Benzin)
1-(2-Amino-5-chlor-benzyl)-imidazol-2-carbonsäureäthylester;
1-(2-Amino-5-methoxy-benzyl)-imidazol-2-carbonsäureäthylester;
1-(2-Amino-4-fluor-benzyl)-imidazol-2-carbonsäureäthylester.

c) *10,11-Dihydro-5H-imidazo[2,1c][1,4]benzodiazepin-11-on*

α) Eine Lösung von 10 g (0,04 Mol) 1-(2-Aminobenzyl)-imidazol-2-carbonsäureäthylester in 70 ml Eisessig wird 3 Stunden unter Rückfluß erhitzt. Nach dem Eindampfen wird der Rückstand in 40 ml Methanol gelöst und die methanolische Lösung bis zur sauren Reaktion mit ätherischer Salzsäure versetzt.

Die anfallenden Kristalle werden abgesaugt, mit Äther gewaschen und getrocknet. Man erhält 8,6 g (89,4 % d. Th.) 10,11-Dihydro-5H-imidazo[2,1-c][1,4]benzodiazepin-11-on-hydrochlorid vom Fp. 315°C (Zers.).

Analog werden erhalten:

8-Chlor-10,11-dihydro-5H-imidazo[2,1-c][1,4]benzodiazepin-11-on-hydrochlorid;
7-Methyl-10,11-dihydro-5H-imidazo[2,1-c][1,4]benzodiazepin-11-on-hydrochlorid; Fp. 314°C (Zers.)
7-Methoxy-10,11-dihydro-5H-imidazo[2,1-c][1,4]-benzodiazepin-11-on-hydrochlorid.

β) 500 mg 1-(2-Aminobenzyl)-imidazol-2-carbonsäureäthylester, 80 mg Kalium-tert.-butylat und 3 ml Dimethylformamid werden unter Rühren 15 Minuten auf 150°C erhitzt. Anschließend wird das Lösungsmittel im Vakuum abdestilliert und der Rückstand mit wenig Wasser und Cyclohexan behandelt. Die hierbei entstehenden Kristalle werden abgesaugt, mit Aceton gewaschen und nach dem Trocknen in Methanol gelöst. Beim Ansäuern der methanolischen Lösung mit ätherischer Salzsäure erhält man 285 mg (62,6 % d. Th.) 10,11-Dihydro-5H-imidazo[2,1-c][1,4]-benzodiazepin-11-on-hydrochlorid vom Fp. 312°C (Zers.).

d) *10,11-Dihydro-5H-imidazo[2,1c][1,4]benzodiazepin-11-thion*

Eine Mischung von 2,35 g (0,01 Mol) 10,11-Dihydro-5h-imidazo[2,1-c][1,4]-benzodiazepin-11-on-hydrochlorid, 0,89 g (0,004 Mol) Phosphorpentasulfid und 25 ml absolutem Pyridin wird unter Rühren 4 Stunden unter Rückfluß erhitzt. Anschließend wird das Pyridin abgedampft und der Rückstand mit 25 ml 5 %-iger Sodalösung und 1 ml Methanol behandelt. Nach längerem Rühren tritt allmählich Kristallisation ein. Die hellbraunen Kristalle werden abgesaugt, mit Wasser gewaschen und getrocknet. Ausbeute 1,95 g (90,7 % d. Th.); Fp. 222 - 227°C. Nach dem Umkristallisieren aus Acetonitril schmilzt die Verbindung bei 227 - 231°C.

Analog wird erhalten:

7-Methoxy-10,11-dihydro-5H-imidazo[2,1-c][1,4]benzodiazepin-11-thion.

e) *11-Methylthio-5H-imidazo[2,1-c][1,4]benzodiazepin*

Eine Lösung von 1,1 g (0,048 Mol) Natrium in 120 ml Äthanol wird unter Rühren mit 10,3 g (0,048 Mol) 10,11-Dihydro-5H-imidazo[2,1-c][1,4]benzodiazepin-11-thion versetzt. Nach 1 Stunde werden bei Raumtemperatur 4,8 ml Methyljodid zugefügt. Allmählich bildet sich ein Niederschlag. Die Suspension wird nach 4 Stunden bei Raumtemperatur gerührt und anschließend das Lösungsmittel im Vakuum abdestilliert. Der Rückstand wird mit Chloroform aufgenommen und mit verdünnter, wäßriger Kaliumcarbonatlösung ausgeschüttelt.

Die organische Phase wird abgetrennt, über wasserfreiem Natriumsulfat getrocknet und im Vakuum eingedampft. Der Rückstand [10 g (91 % d. Th.); Fp. 144 - 150°C] kann ohne zusätzliche Reinigung weiter umgesetzt werden.

Analog wird erhalten:

7-Methoxy-11-methylthio-5H-imidazo[2,1-c][1,4]-benzodiazepin.

Die folgenden Beispiele dienen zur näheren Erläuterung der Erfindung, ohne sie jedoch zu beschränken:

**Beispiel 1**

*11-[4-Methyl-piperazin(1)yl]-5H-imidazo[2,1-c][1,4]benzodiazepin nach Methode a)*

Eine Mischung von 5 g (0,22 Mol) 11-Methylthio-5H-imidazo[2,1-c][1,4]benzodiazepin (Fp. 144 - 150°C), 17,5 g (0,175 Mol) 1-Methyl-piperazin, 10 Tropfen Eisessig und 45 ml Xylol wird 24 Stunden unter Rückfluß erhitzt. Anschließend wird die Reaktionsmischung im Vakuum eingedampft und der Rückstand zwischen Chloroform und verdünnter, wäßriger Kaliumcarbonatlösung verteilt. Die organische Phase wird abgetrennt, mit wasserfreiem Natriumsulfat getrocknet und das Lösungsmittel abdestilliert. Der Rückstand (5,3 g) wird anschließend an Kieselgel mit Methanol chromatographiert. Die reinen Fraktionen werden gesammelt und eingedampft. Der Rückstand wird aus Toluol/Benzin umkristallisiert. Man erhält 2,5 g (40,8 % d. Th.) reines 11-[4-Methyl-piperazin(1)yl]-5H-imidazo[2,1-c] [1,4]benzodiazepin (Fp. 121 - 124°C).

In analoger Weise werden erhalten:

11-(1-Piperazinyl)-5H-imidazo[2,1-c][1,4]benzodiazepin, Fp. 175 - 177°C (Toluol), durch Umsetzung von 11-Methylthio-5H-imidazo[2,1-c][1,4]benzodiazepin mit Piperazin,

7-Methoxy-11-[4-methyl-piperazin-(1)-yl-5H-imidazo [2,1-c][1,4]benzodiazepin durch Umsetzung von 7-Methoxy-11-methylthio-5H-imidazo[2,1-c][1,4]benzodiazepin mit 1-Methyl-piperazin.

**Beispiel 2**

*11-[4-Methyl-piperazin-(1)-yl]-5H-imidazo[2,1-c][1,4] benzodiazepin nach Methode b)*

Zu einer Lösung von 2,5 g (0,01 Mol) 1-(2-Aminobenzyl)-imidazol-2-carbonsäureäthylester in 12 ml 1-Methylpiperazin und 50 ml Anisol wird unter Rühren und Stickstoff eine Lösung von 3 ml Titantetrachlorid in 12 ml Anisol langsam zugetropft. Die Reaktionsmischung wird 1 Stunde unter Rückfluß erhitzt. Nach dem Abkühlen werden die unlöslichen Anteile abgetrennt und die hierbei erhaltene Lösung wird mit 4 %-iger Kaliumcarbonatlösung ausgeschüttelt. Die organische Phase wird mit Wasser gewaschen und nach dem Trocknen mit wasserfreiem Natriumsulfat im Vakuum eingedampft. Der teils kristalline Rückstand (4,2 g) wird mit Benzin gewaschen und getrocknet. Die Ausbeute an reinem 11-[4-Methyl-piperazin-(1)-yl]-5H-imidazo[2,1-c][1,4]benzodiazepin beträgt 2,4 g (83,7 % d. Th.); Fp. 123 - 125°C.

Nach dem gleichen Verfahren werden folgende Verbindungen dargestellt:

8-Chlor-11-[4-methyl-piperazin-(l)-yl]-5H-imidazo[2,1-c] [1,4]benzodiazepin; Fp. 177 - 179°C (Essigester/Benzin)
8-Chlor-11-(1-piperazinyl)-5H-imidazo[2,1-c][1,4]benzodiazepin; Fp. 120 - 123°C (Essigester/Benzin)
7-Methyl-11-[4-methyl-piperazin-(1)-yl]-5H-imidazo [2,1-c][1,4]benzodiazepin; Fp. 170 - 172°C.
11-[4-Benzyl-piperazin-(1)-yl]-5H-imidazo[2,1-c][1,4] benzodiazepin; Fp. 158 - 159°C (Essigester/Benzin)
11-[4-Methyl-homopiperazin-(1)-yl]-5H-imidazo[2,1-c][1,4]benzodiazepin
7-Chlor-11-[4-methyl-piperazin-(1)-yl]-5H-imidazo[2,1-c][1,4]benzodiazepin
8-Fluor-11-[4-methyl-piperazin-(1)-yl]-5H-imidazo[2,1-c][1,4]benzodiazepin
11-[4-Äthyl-piperazin-(1)-yl]-5H-imidazo[2,1-c][1,4] benzodiazepin; Fp. 129 - 130°C.

**Beispiel 3**

*11-[4-Methyl-piperazin-(1)-yl]-5H-imidazo[2,1-c][1,4] benzodiazepin nach Methode a)*

Eine Suspension von 5 g (0,02 Mol) 10,11-Dihydro-5H-imidazo[2,1-c][1,4]benzodiazepin-11-on-hydrochlorid in 70 ml Anisol und 20 ml 1-Methyl-piperazin wird unter Rühren und Stickstoff tropfenweise mit einer Lösung von 6,3 ml Titantetrachlorid in 30 ml Anisol versetzt. Die Reaktionsmischung wird 1 Stunde unter Rückfluß erhitzt und anschließend auf ca. 60°C abgekühlt. Nach dem Abtrennen von unlöslichen Anteilen wird die erkaltete Reaktionslösung mit 50 ml Toluol verdünnt und mit 40 ml gesättigter Kaliumcarbonatlösung ausgeschüttelt. Die organische Phase wird abgetrennt und nach dem Trocknen über wasserfreiem Natriumsulfat im Vakuum eingedampft. Der kristalline Rückstand wird mit Benzin gewaschen. Man erhält 4,7 g (78,7 % d. Th.) 11-[4-Methyl-piperazin-(1)-yl]-5H-imidazo[2,1-c][1,4]benzodiazepin vom Fp. 121 - 123°C.

**Beispiel 4**

*11-[4-allyl-piperazin-(1)-yl]-5H-imidazo[2,1-c][1,4] benzodiazepin nach Methode c)*

Eine Lösung von 2,9 g (0,011 Mol) 11-(1-Piperazinyl)-5H-imidazo[2,1-c][1,4]benzodiazepin in 50 ml Methanol wird mit 1,5 g (0,011 Mol) wasserfreiem Kaliumcarbonat und 1,44 g (0,012 Mol) Allylbromid versetzt. Die Reaktionsmischung wird unter Rühren 1 Stunde auf 60°C erhitzt. Anschließend wird das Lösungsmittel abdestilliert und der Rückstand zwischen Chloroform und Wasser verteilt. Die organische Phase wird nach dem Trocknen über wasserfreiem Natriumsulfat eingeengt. Der Rückstand wird durch Säulenchromatographie (Kieselgel/Methanol) und anschließende Umkristallisation mit Acetonitril gereinigt. Man erhält 1,9 g (57 % d. Th.) 11-[4-Allyl-piperazin-(1)-yl]-5H-imidazo[2,1-c][1,4] benzodiazepin; Fp. 168 - 170°C.

Analog werden hergestellt:

8-Chlor-11-[4-(2-hydroxyäthyl)-piperazin-(1)-yl]-5H-imidazo[2,1-c][1,4]benzodiazepin
8-Chlor-11-[4-cyclopropylmethyl-piperazin-(1)-yl]-5H-imidazo[2,1-c-][1,4]benzodiazepin
8-Chlor-11-[4-propargyl-piperazin-(1)-yl]-5H-imidazo[2,1-c][1,4]benzodiazepin
11-[4-(3-Hydroxypropyl)-piperazin-(1)-yl]-5H-imidazo[2,1-c][1,4]benzodiazepin.

**Pharmazeutische Formulierungsbeispiele**

**a) Dragees**

1 Drageekern enthält:

| | |
|---|---|
| Wirkstoff gemäß vorliegender Erfindung | 50,0 mg |
| Milchzucker | 28,5 mg |
| Maisstärke | 17,0 mg |
| Gelatine | 2,0 mg |
| Magnesiumstearat | 0,5 mg |
| | 98,0 mg |

Herstellung:
Die Mischung der Wirksubstanz mit Milchzucker und Maisstärke wird mit einer 10 %-igen wäßrige Gelatinelösung durch ein Sieb mit 1 mm Maschenweite granuliert, bei 40°C getrocknet und nochmals durch ein Sieb getrieben. Das so erhaltene Granulat wird mit Magnesiumstearat gemischt und verpreßt. Die so erhaltenen Kerne werden in üblicher Weise mit einer Hülle überzogen, die mit Hilfe einer wäßrigen Suspension von Zucker, Titandioxyd, Talkum und Gummi arabicum aufgebracht wird. Die fertigen Dragees werden mit Bienenwachs poliert. Dragee-Endgewicht: 100 mg.

**b) Tabletten**

| | |
|---|---|
| Wirkstoff gemäß vorliegender Erfindung | 20,0 mg |
| Milchzucker | 55,0 mg |
| Maisstärke | 38,0 mg |
| lösliche Stärke | 4,0 mg |
| Magnesiumstearat | 1,0 mg |
| | 118,0 mg |

Herstellung:
Wirkstoff und Magnesiumstearat werden mit einer wäßrigen Lösung der löslichen Stärke granuliert, das Granulat getrocknet und innig mit Milchzucker und Maisstärke vermischt. Das Gemisch wird sodann zu Tabletten von 100 mg Gewicht verpreßt, die 2 mg Wirkstoff enthalten.

**c) Suppositorien**

1 Zäpfchen enthält:

| | |
|---|---|
| Wirkstoff gemäß vorliegender Erfindung | 5,0 mg |
| Zäpfchenmasse | 1695,0 mg |

Herstellung:
Die feingepulverte Substanz wird mit Hilfe eines Eintauch-Homogenisators in die geschmolzene und auf 40°C abgekühlte Zäpfchenmasse eingerührt. Die Masse wird bei 35°C in leicht vorgekühlte Formen gegossen.

**d) Ampullen**

| | |
|---|---|
| Wirkstoff gemäß vorliegender Erfindung | 2,0 mg |
| Natriumchlorid | 18,0 mg |
| destilliertes Wasser | ad 2,0 ml |

Herstellung:
Wirkstoff und Natriumchlorid werden in Wasser gelöst, die Lösung frei von suspendierten Partikeln filtriert und in 2 ccm-Ampullen unter aseptischen Bedingungen abgefüllt. Zuletzt werden die Ampullen sterilisiert und verschlossen. Jede Ampulle enthält 2 mg Wirkstoff.

**e) Inhalationsaerosol**

Das Aerosol ist aus folgenden Bestandteilen zusammengesetzt:

| | |
|---|---|
| Wirkstoff gemäß vorliegender Erfindung | 1,00 Teile |
| Sojalecithin | 0,20 Teile |
| Treibgasmischung (Frigen® 11, 12 und 14) | ad 100,00 Teile |

Herstellung:
Die Bestandteile werden in an sich bekannter Weise zusammengemischt und das Gemisch in Aerosolbehälter gefüllt, die ein Dosierventil enthalten, das pro Betätigung zwischen 5 und 20 mg Wirksubstanz abgibt.

**Patentansprüche**

1. 11-Piperazinyl-5H-imidazo[2,1-c][1,4]benzodiazepine der allgemeinen Formel

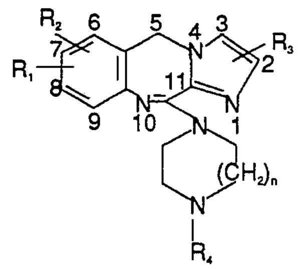

(I)

worin

$R_1$ und $R_2$, die gleich oder verschieden sein können, Wasserstoff, Fluor, Chlor, Brom, Trifluormethyl, Hydroxy, Nitro, Amino, Cyano, Methylsulfonamido, $C_{1-4}$ Alkyl, $C_{1-3}$ Alkoxy, $C_{1-3}$ Alkylthio oder $C_{2-4}$ Acylamino,
$R_3$ Wasserstoff, Fluor, Chlor, Brom oder $C_{1-3}$ Alkyl,

$R_4$ Wasserstoff, $C_{1-4}$ Alkyl, $C_{2-4}$ Hydroxyalkyl, $C_{1-2}$ Alkoxy-$C_{2-4}$ alkyl, $C_{3-6}$ Alkenyl, $C_{3-6}$ Alkinyl, $C_{3-7}$ Cycloalkyl, $C_{3-7}$ Cycloalkyl-$C_{1-3}$ alkyl oder Phenyl-$C_{1-3}$ Alkyl, wobei der Phenylring gegebenenfalls durch Fluor, Chlor, Brom, Methyl, Methoxy, Hydroxy oder Trifluormethyl substituiert sein kann, und
n eine der Zahlen 1 oder 2 bedeutet und deren physiologisch verträgliche Säureadditionssalze.

2. Verbindungen der allgemeinen Formel I gemäß Anspruch 1 sowie deren physiologisch verträgliche Säureadditionssalze, dadurch gekennzeichnet, daß $R_1$ und $R_2$, die gleich oder verschieden sein können, in 7- beziehungsweise 8-Stellung des Moleküls stehen und Wasserstoff oder ein Halogenatom und $R_3$ Wasserstoff bedeutet und daß $R_4$ für eine der oben angegebenen Bedeutungen steht.

3. Verbindungen der allgemeinen Formel I gemäß Anspruch 1 sowie deren physiologisch verträgliche Säureadditionssalze, dadurch gekennzeichnet, daß $R_1$ und $R_2$, die gleich oder verschieden sein können, in 7- beziehungsweise 8-Stellung des Moleküls stehen und Wasserstoff oder ein Halogenatom, $R_3$ Wasserstoff und $R_4$ Wasserstoff oder die Methylgruppe bedeutet.

4. 11-[4-Methyl-piperazin(1)yl]-5H-imidazo[2,1-c][1,4] benzodiazepin

5. 11-(1-Piperazinyl)-5H-imidazo[2,1-c][1,4] benzodiazepin.

6. Als Zwischenprodukte Verbindungen der allgemeinen Formel

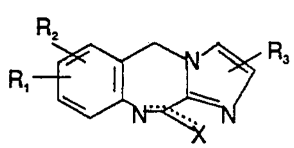

(II)

worin

$R_1$ und $R_2$, die gleich oder verschieden sein können, Wasserstoff, Fluor, Chlor, Brom, Trifluormethyl, Hydroxy, Nitro, Amino, Cyano, Methylsulfonamido, $C_{1-4}$ Alkyl, $C_{1-3}$ Alkoxy, $C_{1-3}$ Alkylthio oder $C_{2-4}$ Acylamino,
$R_3$ Wasserstoff, Fluor, Chlor, Brom oder $C_{1-3}$ Alkyl und
X $C_{1-4}$ Alkoxy, $C_{1-4}$ Alkylmercapto, Chlor, Brom, Sauerstoff und Schwefel bedeutet.

7. Als Zwischenprodukte Verbindungen der allgemeinen Formel

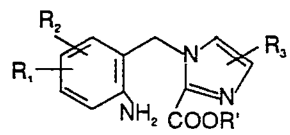

(IV)

worin
$R_1$ und $R_2$, die gleich oder verschieden sein können, Wasserstoff, Fluor, Chlor, Brom, Trifluormethyl, Hydroxy, Nitro, Amino, Cyano, Methylsulfonamido, $C_{1-4}$ Alkyl, $C_{1-3}$ Alkylthio oder $C_{2-4}$ Acylamino,
$R_3$ Wasserstoff, Fluor, Chlor, Brom oder $C_{1-3}$ Alkyl und
R' Wasserstoff oder einen $C_{1-4}$ Alkylrest bedeutet.

8. Verfahren zur Herstellung 11-Piperazinyl-5H-imidazo[2,1-c][1,4]benzodiazepine der allgemeinen Formel

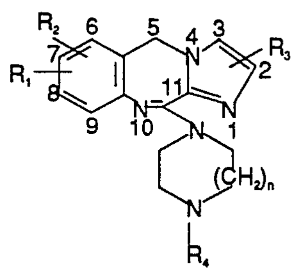

(I)

worin

$R_1$ und $R_2$, die gleich oder verschieden sein können, Wasserstoff, Fluor, Chlor, Brom, Trifluormethyl, Hydroxy, Nitro, Amino, Cyano, Methylsulfonamido, $C_{1-4}$ Alkyl, $C_{1-3}$ Alkoxy, $C_{1-3}$ Alkylthio oder $C_{2-4}$ Acylamino,
$R_3$ Wasserstoff, Fluor, Chlor, Brom oder $C_{1-3}$ Alkyl,
$R_4$ Wasserstoff, $C_{1-4}$ Alkyl, $C_{2-4}$ Hydroxyalkyl, $C_{1-2}$ Alkoxy-$C_{2-4}$ alkyl, $C_{3-6}$ Alkenyl, $C_{3-6}$ Alkinyl, $C_{3-7}$ Cycloalkyl, $C_{3-7}$ Cycloalkyl-$C_{1-3}$ alkyl oder Phenyl-$C_{1-3}$ Alkyl, wobei der Phenylring gegebenenfalls durch Fluor, Chlor, Brom, Methyl, Methoxy, Hydroxy oder Trifluormethyl substituiert sein kann, und
n eine der Zahlen 1 oder 2 bedeutet sowie von deren physiologisch verträglichen Säureadditionssalzen, dadurch gekennzeichnet, daß man

a) eine Verbindung der allgemeinen Formel

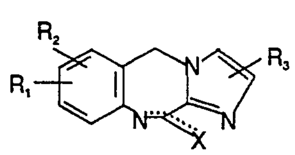

(II)

worin $R_1$, $R_2$ und $R_3$ die oben angeführte Bedeutung besitzen und X Chlor oder Brom, $C_{1-4}$ Alkoxy, $C_{1-4}$ Alkylthio, = O oder = S bedeutet, mit einem Piperazin der allgemeinen Formel

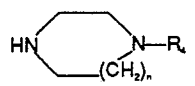

(III)

worin $R_4$ und n die oben angegebene Bedeutung besitzen, umsetzt, oder daß man

b) eine Verbindung der allgemeinen Formel

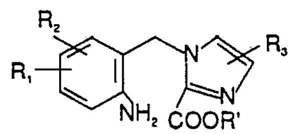

(IV)

worin $R_1$, $R_2$ und $R_3$ die oben angeführte Bedeutung besitzen und R' einen $C_{1-4}$ Alkylrest bedeutet, mit einem Piperazin der allgemeinen Formel III umsetzt, oder daß man

c) eine Verbindung der allgemeinen Formel

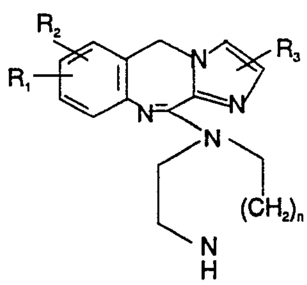

(V)

worin $R_1$, $R_2$, $R_3$ und n die oben angeführte Bedeutung besitzen, mit einer Verbindung der allgemeinen Formel

$R_4$-Z (VI)

worin $R_4$ die oben angeführte Bedeutung mit Ausnahme von Wasserstoff besitzt und Z Chlor, Brom, Jod, Tosyloxy oder Mesyloxy bedeutet, alkyliert, und daß man gegebenenfalls in an sich bekannter Weise ein so erhaltenes Endprodukt der allgemeinen Formel I in ein physiologisch unbedenkliches Säureadditionssalz überführt.

9. Verfahren nach Anspruch 8 a und b, dadurch gekennzeichnet, daß bei Verwendung solcher Ausgangsstoffe der allgemeinen Formel II, in denen X = O bedeutet, und bei Verwendung von Ausgangsstoffen der allgemeinen Formel IV die Reaktion in Gegenwart von Titantetrachlorid in einem geeigneten Lösungsmittel durchgeführt wird.

10. Pharmazeutische Präparate, enthaltend als Wirkstoffe Verbindungen der allgemeinen Formel I in Kombination mit üblichen Hilfs- und/oder Trägerstoffen.

11. Verfahren zur Herstellung von pharmazeutischen Präparaten nach Anspruch 10, dadurch gekennzeichnet, daß man Verbindungen der allgemeinen Formel I mit üblichen galenischen Hilfs- und/oder Trägerstoffen zu üblichen pharmazeutischen Anwendungsformen verarbeitet.

12. Verbindungen der allgemeinen Formel I gemäß Anspruch 1 zur Verwendung für die Herstellung von Arzneimitteln mit antiallergischer Wirkung.

13. Verbindungen der allgemeinen Formel I gemäß Anspruch 1 zur Verwendung für die Herstellung von Arzneimitteln mit neuroleptischer Wirkung.

14. Verwendung von Verbindungen der allgemeinen Formel I gemäß Anspruch 1 zur Herstellung von Arzneimitteln mit analgetischer Wirkung.

15. Verwendung von Verbindungen der allgemeinen Formel I gemäß Anspruch 1 zur Herstellung von Arzneimitteln mit Antihistaminwirkung.

## Claims

1. 11-Piperazinyl-5H-imidazo[2,1-c][1,4]benzodiazepines of general formula

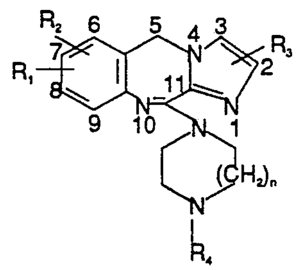

(I)

wherein

$R_1$ and $R_2$, which may be identical or different, represent hydrogen, fluorine, chlorine, bromine, trifluoromethyl, hydroxy, nitro, amino, cyano, methylsulphonamido, $C_{1-4}$ alkyl, $C_{1-3}$ alkoxy, $C_{1-3}$ alkylthio or $C_{2-4}$ acylamino,
$R_3$ represents hydrogen, fluorine, chlorine, bromine or $C_{1-3}$ alkyl,
$R_4$ represents hydrogen, $C_{1-4}$ alkyl, $C_{2-4}$ hydroxyalkyl, $C_{1-2}$ alkoxy-$C_{2-4}$ alkyl, $C_{3-6}$ alkenyl, $C_{3-6}$ alkynyl, $C_{3-7}$ cycloalkyl, $C_{3-7}$ cycloalkyl-$C_{1-3}$ alkyl or phenyl-$C_{1-3}$ alkyl, wherein the phenyl ring may optionally be substituted by fluorine, chlorine, bromine, methyl, methoxy, hydroxy or trifluoromethyl, and
n represents one of the numbers 1 or 2 and the physiologically acceptable acid addition salts thereof.

2. Compounds of general formula I according to claim 1 and the physiologically acceptable acid addition salts thereof, characterised in that $R_1$ and $R_2$, which may be identical or different, are in the 7- and 8-positions of the molecule, and represent hydrogen or a halogen atom and $R_3$ represents hydrogen and $R_4$ has one of the definitions given hereinbefore.

3. Compounds of general formula I according to claim 1 and the physiologically acceptable acid addition salts thereof, characterised in that $R_1$ and $R_2$, which may be identical or different, are in the 7- and 8-positions of the molecule, and represent hydrogen or a halogen atom, $R_3$ represents hydrogen and $R_4$ represents hydrogen or a methyl group.

4. 11-[4-Methyl-piperazin(1)yl]-5H-imidazo[2,1-c][1,4]benzodiazepine.

5. 11-(1-Piperazinyl)-5H-imidazo[2,1-c][1,4]benzodiazepine.

6. As intermediate products, compounds of general formula

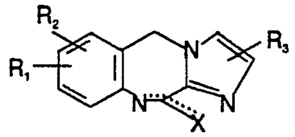

(II)

wherein

$R_1$ and $R_2$, which may be identical or different, represent hydrogen, fluorine, chlorine, bromine, trifluoromethyl, hydroxy, nitro, amino, cyano, methylsulphonamido, $C_{1-4}$ alkyl, $C_{1-3}$ alkoxy, $C_{1-3}$ alkylthio or $C_{2-4}$ acylamino,
$R_3$ represents hydrogen, fluorine, chlorine, bromine or $C_{1-3}$ alkyl and
X represents $C_{1-4}$ alkoxy, $C_{1-4}$ alkylmercapto, chlorine, bromine, oxygen and sulphur.

7. As intermediate products, compounds of general formula

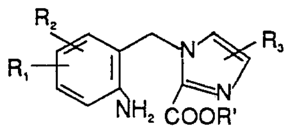

(IV)

wherein

$R_1$ and $R_2$, which may be identical or different, represent hydrogen, fluorine, chlorine, bromine, trifluoromethyl, hydroxy, nitro, amino, cyano, methylsulphonamido, $C_{1-4}$ alkyl, $C_{1-3}$ alkoxy, $C_{1-3}$ alkylthio or $C_{2-4}$ acylamino,
$R_3$ represents hydrogen, fluorine, chlorine, bromine or $C_{1-3}$ alkyl and
R' represents hydrogen or a $C_{1-4}$ alkyl group.

8. Process for preparing 11-piperazinyl-5H-imidazo[2,3-c][1,4]benzodiazepines of general formula

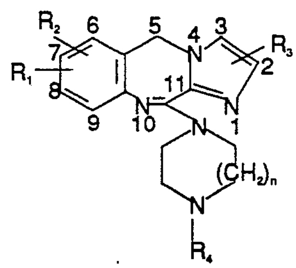

(I)

wherein

$R_1$ and $R_2$, which may be identical or different, represent hydrogen, fluorine, chlorine, bromine, trifluoromethyl, hydroxy, nitro, amino, cyano, methylsulphonamido, $C_{1-4}$ alkyl, $C_{1-3}$ alkoxy, $C_{1-3}$ alkylthio or $C_{2-4}$ acylamino,
$R_3$ represents hydrogen, fluorine, chlorine, bromine or $C_{1-3}$ alkyl,
$R_4$ represents hydrogen: $C_{1-4}$ alkyl, $C_{2-4}$ hydroxyalkyl, $C_{1-2}$ alkoxy-$C_{2-4}$ alkyl, $C_{3-6}$ alkenyl, $C_{3-6}$ alkynyl, $C_{3-7}$ cycloalkyl, $C_{3-7}$ cycloalkyl-$C_{1-3}$ alkyl or phenyl-$C_{1-3}$ alkyl, wherein the phenyl ring may optionally be substituted by fluorine, chlorine, bromine, methyl, methoxy, hydroxy or trifluoromethyl, and
n represents one of the numbers 1 or 2 and the physiologically acceptable acid addition salts thereof, characterised in that

a) a compound of general formula

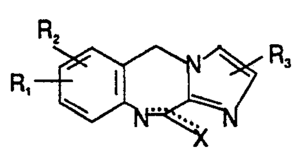

(II)

wherein $R_1$, $R_2$ and $R_3$ are defined as hereinbefore and X represents chlorine or bromine, $C_{1-4}$ alkoxy, $C_{1-4}$ alkylthio, = O or = S, is reacted with a piperazine of general formula

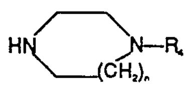

(III)

wherein $R_4$ and n are defined as hereinbefore, or

b) a compound of general formula

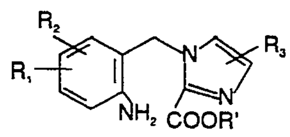

(IV)

wherein $R_1$, $R_2$ and $R_3$ are defined as hereinbefore and R' represents a $C_{1-4}$ alkyl group, is reacted with a piperazine of general formula III, or

c) a compound of general formula

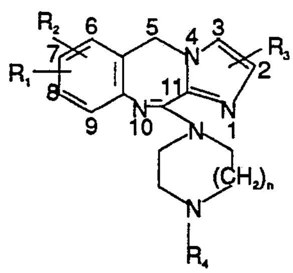

(V)

wherein $R_1$, $R_2$, $R_3$ and n are defined as hereinbefore, is alkylated with a compound of general formula

$R_4$-Z (VI)

wherein $R_4$ has the meanings given hereinbefore with the exception of hydrogen and Z represents chlorine, bromine, iodine, tosyloxy or mesyloxy, and if desired an end product of general formula I thus obtained is converted into a physiologically harmless acid addition salt in a manner known *per se*.

9. Process according to claim 8a or b, characterised in that when starting materials of general formula II are used wherein X = O, and when starting materials of general formula IV are used, the reaction is carried out in the presence of titanium tetrachloride in a suitable solvent.

10. Pharmaceutical preparations containing as active substances compounds of general formula I in conjunction with excipients and/or carriers.

11. Process for preparing pharmaceutical preparations according to claim 10, characterised in that compounds of general formula I are processed with conventional galenic excipients and/or carriers to obtain conventional pharmaceutical forms for administration.

12. Compounds of general formula I according to claim 1 for use in the preparation of pharmaceutical compositions with an anti-allergic activity.

13. Compounds of general formula I according to claim 1 for use in the preparation of pharmaceutical compositions with a neuroleptic activity.

14. Compounds of general formula I according to claim 1 for use in the preparation of pharmaceutical compositions with an analgesic activity.

15. Compounds of general formula I according to claim 1 for use in the preparation of pharmaceutical compositions with an antihistamine activity.

**Revendications**

1. 11-pipérazinyl-5H-imidazo[2,1-c][1,4]benzodiazépines de formule générale

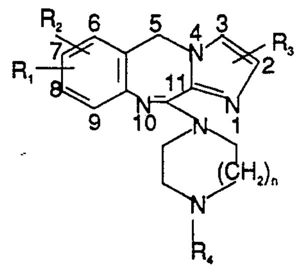

(I)

dans laquelle

$R_1$ et $R_2$, qui peuvent être identiques ou différents, représentent hydrogène, fluor, chlore, brome, trifluorométhyle, hydroxy, nitro, amino, cyano, méthylsulfonamido, alcoyle en $C_{1-4}$, alcoxy en $C_{1-3}$, alcoylthio en $C_{1-3}$ ou acylamino en $C_{2-4}$,
$R_3$ représente hydrogène, fluor, chlore, brome ou alcoyle en $C_{1-3}$,
$R_4$ représente hydrogène, alcoyle en $C_{1-4}$, hydroxyalcoyle en $C_{2-4}$, alcoxy en $C_{1-2}$-alcoyle en $C_{2-4}$, alcényle en $C_{3-6}$, alcynyle en $C_{3-6}$, cycloalcoyle en $C_{3-7}$, cycloalcoyl en $C_{3-7}$-alcoyle en $C_{1-3}$ ou phényl-alcoyle en $C_{1-3}$, le cycle phényle pouvant être éventuellement substitué par fluor, chlore, brome, méthyle, méthoxy, hydroxy ou trifluorométhyle, et
n représente l'un des nombres 1 ou 2 et leurs sels d'addition d'acides physiologiquement supportables.

2. Composés de formule générale I selon la revendication 1 ainsi que leurs sels d'addition d'acides physiologiquement supportables, caractérisés en ce que $R_1$ et $R_2$, qui peuvent être identiques ou différents, sont en position 7 ou respectivement 8 de la molécule et représentent hydrogène ou un atome d'halogène et $R_3$ représente hydrogène et en ce que $R_4$ remplace l'une des significations données plus haut.

3. Composés de formule générale I selon la revendication 1 ainsi que leurs sels d'addition d'acides physiologiquement supportables, caractérisés en ce que $R_1$ et $R_2$, qui peuvent être identiques ou différents, sont en position 7 ou respectivement 8 de la molécule et représentent hydrogène ou un atome d'halogène, $R_3$ représente hydrogène et $R_4$ représente hydrogène ou le groupe méthyle.

4. La 11-[4-méthyl-pipérazine(1)yl]-5H-imidazo[2,1-c][1,4]benzodiazépine.

5. La 11-(1-pipérazinyl)-5H-imidazo[2,1-c][1,4]benzodiazépine.

6. En tant que produits intermédiaires, les composés de formule générale

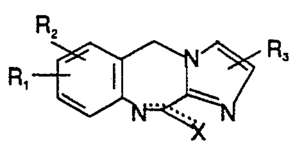

(II)

dans laquelle

$R_1$ et $R_2$, qui peuvent être identiques ou différents, représentent hydrogène, fluor, chlore, brome, trifluorométhyle, hydroxy, nitro, amino, cyano, méthylsulfonamido, alcoyle en $C_{1-4}$, alcoxy en $C_{1-3}$, alcoylthio en $C_{1-3}$ ou acylamino en $C_{2-4}$,
$R_3$ représente hydrogène, fluor, chlore, brome ou alcoyle en $C_{1-3}$ et
X représente alcoxy en $C_{1-4}$, alcoylmercapto en $C_{1-4}$, chlore, brome, oxygène et soufre.

7. En tant que produits intermédiaires, les composés de formule générale

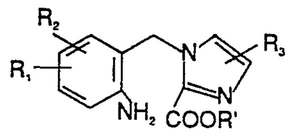

(IV)

dans laquelle

$R_1$ et $R_2$, qui peuvent être identiques ou différents, représentent hydrogène, fluor, chlore, brome, trifluorométhyle, hydroxy, nitro, amino, cyano, méthylsulfonamido, alcoyle en $C_{1-4}$, alcoxy en $C_{1-3}$, alcoylthio en $C_{1-3}$ ou acylamino en $C_{2-4}$,
$R_3$ représente hydrogène, fluor, chlore, brome ou alcoyle en $C_{1-3}$ et
R' représente hydrogène ou un radical alcoyle en $C_{1-4}$.

8. Procédé pour la préparation des 11-pipérazinyl-5H-imidazo[2,1-c][1,4]benzodiazépines de formule générale

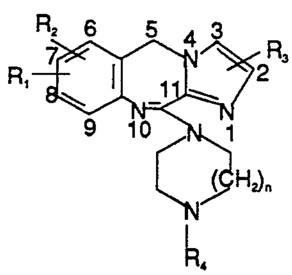

(I)

dans laquelle

$R_1$ et $R_2$, qui peuvent être identiques ou différents, représentent hydrogène, fluor, chlore, brome, trifluorométhyle, hydroxy, nitro, amino, cyano, méthylsulfonamido, alcoyle en $C_{1-4}$, alcoxy en $C_{1-3}$, alcoylthio en $C_{1-3}$ ou acylamino en $C_{2-4}$,
$R_3$ représente hydrogène, fluor, chlore, brome ou alcoyle en $C_{1-3}$,
$R_4$ représente hydrogène, alcoyle en $C_{1-4}$, hydroxyalcoyle en $C_{2-4}$, alcoxy en $C_{1-2}$-alcoyle en $C_{2-4}$, alcényle en $C_{3-6}$, alcynyle en $C_{3-6}$, cycloalcoyle en $C_{3-7}$, cycloalcoyl en $C_{3-7}$-alcoyle en $C_{1-3}$ ou phényl-alcoyle en $C_{1-3}$, le cycle phényle pouvant être éventuellement substitué par fluor, chlore, brome, méthyle, méthoxy, hydroxy ou trifluorométhyle, et
n représente l'un des nombres 1 ou 2 ainsi que leurs sels d'addition d'acides physiologiquement supportables, caractérisé en ce que

a) on fait réagir un composé de formule générale

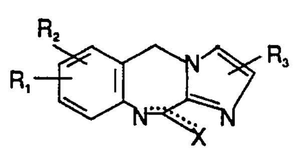

(II)

dans laquelle $R_1$, $R_2$ et $R_3$ possèdent la signification indiquée plus haut et X représente chlore ou brome, alcoxy $C_{1-4}$, alcoylthio en $C_{1-4}$, = O ou = S, avec une pipérazine de formule générale

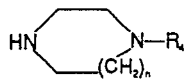

(III)

dans laquelle $R_4$ et n possèdent la signification indiquée plus haut, ou en ce que

b) on fait réagir un composé de formule générale

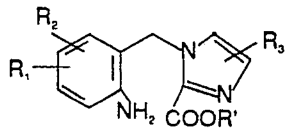

(IV)

dans laquelle $R_1$, $R_2$ et $R_3$ possèdent la signification indiquée plus haut et R' représente un radical alcoyle en $C_{1-4}$, avec une pipérazine de formule générale III, ou en ce que

c) on alcoyle un composé de formule générale

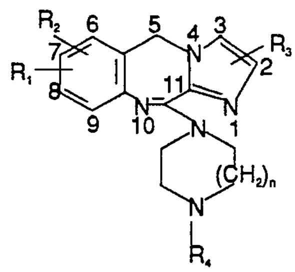

(V)

dans laquelle $R_1$, $R_2$, $R_3$ et n possèdent la signification indiquée au début, au moyen d'un composé de formule générale

$R_4$-Z (VI)

dans laquelle $R_4$ possède la signification indiquée plus haut à l'exception de l'hydrogène et Z signifie chlore, brome, iode, tosyloxy ou mésyloxy, et en ce que éventuellement on transforme, de manière connue en soi, un produit final de formule générale I ainsi obtenu en un sel d'addition d'acide physiologiquement inoffensif.

9. Procédé selon la revendication 8 a) et b), caractérisé en ce que, lors de l'utilisation de matières de départ de formule générale II dans lesquelles X représente = O, et dans le cas de l'utilisation de matières de départ de formule générale IV la réaction est effectuée en présence de tétrachlorure de titane dans un solvant approprié.

10. Préparations pharmaceutiques contenant, en tant que substances actives, des composés de formule générale I en combinaison avec des adjuvants et/ou excipients usuels.

11. Procédé pour la fabrication de préparations pharmaceutiques selon la revendication 10, caractérisé en ce que l'on transforme des composés de formule générale I, au moyen d'adjuvants et/ou excipients galéniques usuels, en formes d'utilisations pharmaceutiques usuelles.

12. Composés de formule générale I selon la revendication 1 pour l'utilisation pour la fabrication de médicaments à activité anti-allergique.

13. Composés de formule générale I selon la revendication 1 pour l'utilisation pour la fabrication de médicaments à activité neuroleptique.

14. Utilisation de composés de formule générale I selon la revendication 1 pour la fabrication de médicaments à activité analgésique.

15. Utilisation de composés de formule générale I selon la revendication 1 pour la fabrication de médicaments à activité anti-histaminique.